(19) Europäisches Patentamt

European Patent Office

Office européen des brevets

(11) **EP 1 132 364 B1**

(12) **EUROPEAN PATENT SPECIFICATION**

(45) Date of publication and mention
of the grant of the patent:
**11.06.2003 Bulletin 2003/24**

(51) Int Cl.⁷: **C07C 17/20**, C07C 17/383,
C07C 19/08

(21) Application number: **01104604.2**

(22) Date of filing: **23.02.2001**

(54) **Process for the preparation of high purity HFC-125**

Verfahren zur Herstellung von hochreinem HFC-125

Procédé pour la préparation de HCF-125 de haute pureté

(84) Designated Contracting States:
**DE ES FR GB IT**

(30) Priority: **09.03.2000 IT MI000474**

(43) Date of publication of application:
**12.09.2001 Bulletin 2001/37**

(73) Proprietor: **Solvay Solexis S.p.A.**
**20121 Milano (IT)**

(72) Inventor: **Cuzzato, Paolo**
**31050 Ponzano Veneto, Treviso (IT)**

(74) Representative: **Jacques, Philippe et al**
**Solvay S.A.**
**Département Propriété Industrielle**
**310, rue de Ransbeek**
**1120 Bruxelles (BE)**

(56) References cited:
**EP-A- 0 356 892** **EP-A- 0 569 832**
**EP-A- 1 070 696**

**Description**

The present invention relates to a process for the production of high purity HFC-125 (pentafluoroethane, $C_2HF_5$). Specifically it relates to the production of HFC-125 containing an amount by moles of CFC-115 (chloropentafluoroethane, $C_2F_5Cl$) lower than 100 ppm, preferably lower than 50 ppm.

It is known in the prior art the need to have available efficient processes for the synthesis of HFC-125, which is a hydrofluorocarbon able to replace some of the chlorofluorocarbons dangerous to the stratospheric ozone. In fact being free from chlorine atoms, HFC-125 has no dangerous effect on the atmospheric ozone, as required by the Montreal Protocol and subsequent amendments. It is also known that the industrial uses of HFC-125 require a very high purity of the product itself.

Two industrially feasible processes for the synthesis of HFC-125 are known. The former process consists in the fluorination with HF of a suitable precursor, in the presence of a fluorination catalyst. Among the compounds from which HFC-125 is obtainable by fluorination with HF, perchloroethylene (PCE) or any compound of general formula $C_2HX_5$, wherein X is any combination of halogens of which at least one different from F, can be mentioned. However perchloroethylene is preferably used since available on the market at industrially acceptable prices.

The latter process comprises the dismutation of HCFC-124 (chlorotetrafluoroethane, $C_2HF_4Cl$) to HFC-125 and HCFC-123 (dichlorotrifluoroethane, $C_2HF_3Cl_2$).

The former process is more direct than the latter, but it has with lower selectivity: in particular it is impossible to obtain by fluorination a high yield of high purity HFC-125, especially as regards the CFC-115 content. This last compound is an undesirable contaminant and very difficult to be separated by distillation, since it forms with HFC-125 an azeotropic mixture. In particular, if one works at high temperatures there is a good productivity of HFC-125, but the process has a low selectivity since also a significant amount of CFC-115 is formed. If, on the contrary, a high selectivity is desired, it is necessary to moderate the reaction conditions to such an extent, in particular by operating at low temperature, wherefore amounts of HFC-125 are obtained which are insufficient to make the process industrially advantageous. See for example USP 5,334,787.

When the HCFC-124 dismutation is instead used, it is necessary to first prepare the reactant HCFC-124 by fluorination of the same above precursors (in particular perchloroethylene) and to subject it to dismutation under suitable conditions in order to have the desired selectivity. Also in this case, in fact, the reaction conditions cannot be changed in order to increase the HCFC-124 conversion, for example by increasing the temperature, since CFC-115 and other by-products are formed as well, even though in a lower amount with respect to the direct fluorination. See for example USP 5,841,006 in the name of the Applicant.

Alternatively, it is possible to subject HFC-125 contaminated by CFC-115 to various treatments to eliminate CFC-115, for example by transforming it into useful products or easily separable from HFC-125. For this purpose, various purification processes of HFC-125 are described in the prior art. In EP 687,660 a process wherein CFC-115 is hydrogenated to HFC-125 with $H_2$ in gaseous phase on a hydrogenation catalyst, is described. As it is known, these reactions require a specific plant able to use gaseous hydrogen, a dangerous reactant due to its flammability and explosiveness, having also a destroying action on various metal materials.

In EP 508,631 the same reduction of CFC-115 to HFC-125 is carried out in liquid phase by using metal hydrides: this route is even less cheap than the previous one, especially because of the high cost of the hydrides, and shows risks of the same type, since hydrides can easily release gaseous hydrogen if they come into contact with humidity traces.

In USP 5,346,595 a multistage distillation process to remove CFC-115 from HFC-125 is described: however not only said process is expensive both as regards the construction of the distillation section and of energetic consumptions associated thereto, but also its effectiveness is poor, since the process reaches at most a HFC-125 purity equal to 99.8%, much lower than the desirable one.

Another method, described in EP 612,709, is the CFC-115 fluorination to perfluoroethane (FC-116, $C_2F_6$) with HF in the presence of chromium-based catalysts. FC-116 is then easily separable from HFC-125. This reaction is however difficult and requires very hard reaction conditions, in particular a high reaction temperature.

Therefore each post-treatment described in the prior art requires a specific step and/or plant, additional costs and in some cases it shows risks for the safety.

The need was therefore felt to produce HFC-125 having a very good purity degree by a simple, cheap, riskless process without requiring an additional step and/or plant.

The Applicant has surprisingly found a process having the above mentioned advantages for the production of HFC-125 having an excellent purity degree, in particular containing an amount by moles of CFC-115 lower than 100 ppm.

An object of the present invention is a process in gaseous phase for the production of HFC-125 comprising the following steps:

- feeding a mixture of HCFC-124 and HFC-134a on a heterogeneous catalyst;
- separation of the HFC-125 from the other reaction products by distillation;

wherein:

the heterogeneous catalyst is selected from the fluorination catalysts of halogenated hydrocarbons.

Preferably the molar ratio of the reactants 134a/124 is higher than 0.6.

The fluorination catalysts which can be mentioned are oxides, oxy-fluorides, oxy-chlorides, chromium, cobalt, nickel fluorides or mixtures thereof. The compounds of trivalent chromium such as oxides, oxy-fluorides, oxy-chlorides, fluorides or mixtures thereof, optionally supported on $AlF_3$, fluorinated alumina or carbon are preferably used.

In the process of the present invention the Applicant has surprisingly and unexpectedly found that the following reaction takes place:

$$C_2ClHF_4 + CF_3CH_2F \rightarrow C_2HF_5 + CF_3CH_2Cl$$

$$(HCFC\text{-}124) + (HFC\text{-}134a) \rightarrow (HFC\text{-}125) + (HCFC\text{-}133a)$$

It has been surprisingly found that the disproportionation reaction of HCFC-124 to HFC-125 and HCFC-123 results strongly inhibited and minimum amounts of CFC-115 and HCFC-123 ($C_2Cl_2HF_3$ isomers) are formed.

The distillation step of the reaction products is easily carried out since there are no azeotropic mixtures wherefore HFC-125 easily separates. At the end of the distillation HFC-125 having a high purity is obtained since it contains an amount by moles of CFC-115 lower than 100 ppm. Therefore the process of the invention results surprisingly improved with respect to the processes described in the prior art.

The process of the present invention is carried out by flowing the mixture 134a/124, optionally diluted with an inert gas, on the catalyst in a fixed or fluidized bed. When the catalyst is used in a fluidized bed, the catalyst particles have granulometry suitable for this kind of use.

Preferably, but not necessarily, the catalyst is dried before the reaction starting by heating (300°-400°C) in inert gas flow, followed by a treatment with anhydrous (pure or diluted) HF at the same temperature. This step can be directly carried out in the reactor used for the process of the invention.

The reaction temperature is in the range 150°-250°C, preferably 180°C-220°. At too low temperatures there is a poor conversion of the reactants; at too high temperatures, organic product decomposition and catalyst deactivation can occurr.

The ratio between the amounts of HFC-134a and HCFC-124 fed into the reactor is an important parameter. If the molar ratio 134a/124 is lower than 0.6, a significant amount of HCFC-124 reacts by disproportionation, producing HCFC-123 and lowering threfore the process selectivity. Besides, if 134a is insufficient also a larger amount of CFC-115 is formed. The higher limit of the molar ratio 134a/124 is not particularly critical, also ratios 134a/124 up to 1,000 can be used. However, when said molar ratio is high, the amount of the fed HCFC-124 decreases and therefore the productivity in HFC-125. The molar ratio 134a/124 is selected in connection with the reaction temperature. The higher the temperature is, the higher said ratio must be in order to have a good selectivity. Preferably the molar ratio 134a/124 of the fed reactants is in the range 0.6-2.

The contact time is not particularly critical: contact times in the range 1-30 seconds, preferably 3-15 seconds are generally used. Also the pressure is not critical: atmospheric pressure or higher, for example also of 10 bar, are commonly used.

The fluorination catalyst used in the process of the present invention is known in the prior art. Among the compounds of trivalent chromium, $Cr_2O_3$ is preferably used, optionally supported, preferably on $AlF_3$. Said catalyst is described in USP 5,345,014 in the name of the Applicant.

The support $AlF_3$ is obtainable by fluorination with anhydrous HF of hydrated aluminum oxide (alumina), preferably in the crystalline form of bohemite or pseudo-bohemite, optionally added of small amounts (preferably up to 5% by weight) of silica. The fluorination is continued up to a fluorine content not lower than 90%, preferably not lower than 95% of the stoichiometric value with respect to $AlF_3$. Preferably the aluminum fluoride is mainly in gamma phase, as described in FR 1,383,927, and it has a surface area generally in the range 25-35 $m^2$/g.

The supported catalyst is preferably prepared by the method known in the prior art as "dry impregnation" (incipient wetness) of the support $AlF_3$ with an aqueous solution of a soluble chromium salt. According to this method the impregnation is carried out by pouring on the support, in sequence, portions of an impregnating solution, such that the volume of each portion is not higher than the volume of the aluminum fluoride pores. The solution for the impregnation is prepared by dissolving in water the required amounts of the corresponding trivalent chromium salts, preferably chlorides. The solution is poured in portions on the support, drying at 110°C for some hours after each addition, to evaporate the water from the support pores. The chromium amount in the supported catalyst is generally in the range 5-15% by weight, preferably 10-15% by weight, as metal amount based on the weight of the supported catalyst.

when the catalyst is not supported, it is prepared by known methods in the prior art, for example by precipitation of a soluble metal salt, prferably of trivalent chromium as described in USP 5,345,014. Among non supported catalysts, commercial products formed by chromium (III) oxide under the form of pellets, optionally added of optional substances, inert under the reaction conditions, having the purpose to improve the mechanical properties thereof, can be mentioned. Carbon can for example be mentioned. These optional components are generally used in amounts of some % by weight.

During the use the catalyst undergoes a slow deactivation, due to the deposit of organic substance. It can be regenerated for example by treatment with air at a temperature in the range 300°C-400°C for 4-8 hours and then with anhydrous HF at 360°C for 12-24 hours.

The following Examples are mentioned for illustrative, but not limitative purposes of the scope of the invention.

**EXAMPLES**

In the Examples 1-8 the used catalyst is a fluorination catalyst containing 10% by weight of chromium on an aluminum fluoride support having a granulometry suitable to the use in a fluidized bed, prepared according to the procedure described in Example 1 of USP 5,841,006 in the name of the Applicant and by using a chromium salt amount such as to have on the final catalyst 10% by weight of chromium. The catalyst of Examples 9-10 is a commercial chromium oxide (Engelhard E-410T®) in the form of pellets of 3x3 mm.

In all the Examples the organic reactants are industrial products having a high purity degree, the analyses are carried out by GLC using a chromatograph equipped with thermoconductivity or flame detector and equipped with a column constituted by perfluoropolyethers on inert support (Fluorcol® or similar support). The reaction reactor is an Inconel®600 tube having diameter 9 mm, equipped with frit at the base and electrically heated, in which the preheated and premixed reactants/diluents are introduced from the top, then the reaction products are bubbled in a water bottle to wash possible acidity traces, and sampled with a syringe for gas. The washing water can be analyzed to titrate the total acidity and the chlorides in case originated from the decomposition of the organic products.

EXAMPLE 1

In the above described reactor, 3.5 of the previously described catalyst are introduced. After a pre-treatment with nitrogen and then with a mixture in 1/1 ratio nitrogen/HF, at the temperature of 360°C, a mixture formed by 5.0 cc/min of HCFC-124, 4.0 cc/min. of HFC-134a and about 10 cc/min. of helium, is fed into the reactor at atmospheric pressure and at the temperature of 210°C. The molar ratio 134a/124 is equal to 0.8.

The contact time meant as the ratio between the catalytic bed volume and the fed gas volume, at the reaction temperature and at atmospheric pressure, is of 5.4 seconds.

The gases coming out from the reactor are analyzed by GLC with thermoconductivity detector. The following analysis is representative of the obtained results:

| HFC-125 | 39.4 (% by moles) |
|---------|-------------------|
| HFC-134a | 3.5 |
| HCFC-124 | 14.3 |
| HCFC-133a | 41.0 |
| HCFC-123 | 1.8 |
| Others | negligible |

HCFC-123 and HCFC-124 are mixtures of the possible isomers, mainly of the symmetric forms $CF_3$-$CHCl_2$ and $CF_3$-CHClF, respectively.

The HCFC-124 conversion is equal to 77.2 % and the productivity in HFC-125 per hour is of about 300 g per Kg of catalyst.

The selectivity (considering HCFC-123 as recyclable product) is unitary within the sensitivity of the used analysis method, as it appears from the fact that no byproduct can be quantified.

The analysis is then repeated by an instrument equipped with flame detector (FID) to determine the content by moles of CFC-115, which results equal to 13.7 ppm referred to the produced HFC-125.

EXAMPLE 2

In the same reactor and under the same conditions, the HCFC-124 and 134a flow-rates are increased respectively to 8.3 and 8.1 cc/min, without dilution with helium. The molar ratio 134a/124 is therefore equal to 0.97 and the contact time is of 6.2 sec.

The following analysis is representative:

| HFC-125 | 28.4 (% by moles) |
|---|---|
| HFC-134a | 19.4 |
| HCFC-124 | 21.3 |
| HCFC-133a | 30.2 |
| HCFC-123 | 0.8 |
| Others | neglegible |

The HCFC-124 conversion is equal to 60.5% and the productivity in HFC-125 is of about 400 g per Kg of catalyst and per hour. The CFC-115 analysis gives 9.2 ppm referred to the produced HFC-125.

EXAMPLE 3

Under the same conditions of the previous Example, a mixture strongly lacking in 134a, having a molar ratio 134a/124 equal to 0.6 and formed by 6.0 cc/min of HCFC-124, 3.6 cc/min of 134a and 13.2 cc/min of helium, is fed. The contact time is of 4.5 sec.

The following analysis is representative:

| HFC-125 | 41.0 (% by moles) |
|---|---|
| HFC-134a | 0.5 |
| HCFC-124 | 15.4 |
| HCFC-133a | 35.7 |
| HCFC-123 | 7.1 |
| Others | 0.3 |

The HCFC-124 conversion is equal to 76.4% and the productivity in HFC-125 is of about 330 g per Kg of catalyst and per hour. The CFC-115 analysis gives 39 ppm referred to the produced HFC-125.

As it can be seen, by operating in lack of 134a the selectivity decreases: CFC-115 increases, other by-products appear and meaningful amounts of HCFC-123 are formed formed.

EXAMPLE 4

10.0 g of the same catalyst are introduced into the reactor of the previous Examples. At the temperature of 180°C a mixture lacking in 134a having a molar ratio 134a/124 equal to 0.68 and formed by 7.5 cc/min of 124, 5.1 cc/min of 134a, 13.1 cc/min of helium,is fed. The contact time is equal to 12.0 sec.

The following analysis is representative:

| HFC-125 | 30.7 (% by moles) |
|---|---|
| HFC-134a | 10.3 |
| HCFC-124 | 27.4 |
| HCFC-133a | 30.0 |
| HCFC-123 | 1.6 |
| Others | neglegible |

The HCFC-124 conversion is equal to 61.9% and the productivity in HFC-125 is of about 115 g per Kg of catalyst. The CFC-115 analysis gives 5.2 ppm referred to the produced HFC-125.

As it can be seen, it is possible to recover selectivity by lowering the temperature, of coarse to the detriment of the productivity.

EXAMPLE 5

A mixture having a molar ratio 134a/124 equal to 0.91 and formed by 6.8 cc of 124 and of 6.2 cc of 134a is fed into the same reactor and on the same catalyst of Example 4, at the same temperature and with the same contact time.

The following analysis is representative:

| HFC-125 | 20.0 (% by moles) |
|---|---|
| HFC-134a | 27.9 |
| HCFC-124 | 31.7 |
| HCFC-133a | 19.6 |
| HCFC-123 | 0.8 |
| Others | negligible |

The HCFC-124 conversion is equal to 39.6% and the productivity in HFC-125 is of about 80 g/h. CFC-115 has not been quantified since lower than the measurement limits, therefore < 1 ppm.

EXAMPLE 6

A mixture having a molar ratio 134a/124 equal to 1.28 and formed by 6.4 cc/min. of 124 and 8.2 of 134a, diluted with 13.7 cc/min. of helium, is fed into the same reactor and on the same catalyst of Example 5, at the temperature of 220°C. The contact time is equal to 10.1 seconds.

The following analysis is representative:

| HFC-125 | 43.2 (% by moles) |
|---|---|
| HFC-134a | 11.6 |
| HCFC-124 | 0.3 |
| HCFC-133a | 44.9 |
| HCFC-123 | <0.1 |
| Others | negligible |

The HCFC-124 conversion is equal to 99.2% and the productivity in HFC-125 is equal to 190 g per Kg of catalyst and per hour. The CFC-115 analysis gives 21 ppm referred to the produced HFC-125.

As it can be seen, at a sufficiently high temperature the HCFC-124 conversion is almost quantitative.

EXAMPLE 7

Under the same conditions of the previous Example, a mixture having a molar ratio 134a/124 equal to 1.51, formed by 16.7 cc/min. of 124, 25.3 cc/min. of 134a and 12.5 cc/min. of helium is fed. The contact time is of 5.2 seconds.

The following analysis is representative:

| HFC-125 | 37.9 (% by moles) |
|---|---|
| HFC-134a | 18.9 |
| HCFC-124 | 1.8 |
| HCFC-133a | 41.3 |
| HCFC-123 | 0.1 |
| Others | negligible |

The HCFC-124 conversion is equal to 95.4% and the productivity in HFC-125 is equal to 480 g per Kg of catalyst and per hour. The CFC-115 analysis gives 7.4 ppm referred to the the produced HFC-125.

EXAMPLE 8

Under the same conditions of the previous Example a mixture having a molar ratio 134a/124 equal to 1.48 and formed by 20.8 cc/min. of 124, 30.8 cc/min. of 134a and 12,9 cc/min. of helium, is fed. The contact time is of 4.4 sec.

The following analysis is representative:

| HFC-125 | 34.6 (% by moles) |
|---|---|

(continued)

| HFC-134a | 20.6 |
|---|---|
| HCFC-124 | 5.5 |
| HCFC-133a | 39.1 |
| HCFC-123 | 0.2 |
| Others | negligible |

The 124 conversion is equal to 86.3% and the productivity in HFC-125 is equal to 540 g per kg of catalyst and per hour. The CFC-115 analysis gives 4.9 ppm referred to the produced HFC-125.

EXAMPLE 9

In the same reactor of the previous Examples 3.5 g of a catalyst are fed formed by chromium oxide Engelhard E-410T® in pellets, which are activated as described in Example 1.
At the temperature of 240°C, a mixture having a molar ratio 134a/124 equal to 0.88 and formed by 8.9 cc/min. of 124 and 7.9 cc/min. of 134a, is fed. The contact time is equal to 5.4 seconds.
The following analysis is representative:

| HFC-125 | 7.8 (% by moles) |
|---|---|
| HFC-134a | 36.7 |
| HCFC-124 | 45.1 |
| HCFC-133a | 7.9 |
| HCFC-123 | 0.3 |
| Others | negligible |

The HCFC-124 conversion is equal to 15.1% and the productivity in HFC-125 is equal to 110 g per kg of catalyst and per hour. The CFC-115 amount in the reaction products results lower than the analytical limits, therefore lower than 1 ppm.

EXAMPLE 10 (comparative)

8.9 cc/min. of HCFC-124 in absence of HFC-134a, are fed into the same reactor and on the same catalyst of Example 9, at the temperature of 240°C. The contact time is of 10.2 seconds.
The following analysis is representative:

| HFC-125 | 19.9 (% by moles) |
|---|---|
| HCFC-124 | 61.4 |
| HCFC-123 | 18.6 |
| Others | 0.2 |

The HCFC-124 conversion is equal to 38.5% and the productivity in HFC-125 is equal to 150 g per kg of catalyst and per hour. The CFC-115 analysis gives about 190 ppm referred to the produced HFC-125.
As it can be seen, in absence of 134a, the HCFC-124 disproportionation takes place, which leads to an unacceptable formation of CFC-115. Besides it is to be noticed how even though the HCFC-124 conversion is more than doubled, due to the increased contact time, the productivity in HFC-125 increases a little, since half of the converted HCFC-124 changes into HCFC-123.

**Claims**

1. A process in gaseous phase for the production of HFC-125 comprising the following steps:

   - feeding a mixture of HCFC-124 and HFC-134a on a heterogeneous catalyst;
   - separation of the HFC-125 from the other reaction products by distillation;

wherein:
the heterogeneous catalyst is selected from fluorination catalysts of halogenated hydrocarbons.

2. A process according to claim 1, wherein the molar ratio of the reactants 134a/124 is higher than 0.6.

3. A process accoarding to claims 1-2, wherein the fluorination catalyst is selected from oxides, oxy-fluorides, oxy-chlorides, chromium, cobalt, nickel fluorides or mixtures thereof.

4. A process according to claim 3, wherein the fluorination catalyst is selected from oxides, oxy-fluorides, oxy-chlorides, trivalent chromium fluorides or mixtures thereof, optionally supported on $AlF_3$, fluorinated alumina or carbon.

5. A process according to claim 4, wherein the fluorination catalyst is $Cr_2O_3$, optionally supported on $AlF_3$.

6. A process according to claims 1-5, wherein the fluorination catalyst is dried before the reaction beginning by heating (300°-400°C) in inert gas flow, followed by a treatment with anhydrous (pure or diluted) HF at the same temperature.

7. A process according to claims 1-6, wherein the molar ratio 134a/124 of the fed reactants is in the range 0.6-2.

8. A process according to claims 1-7, wherein the reaction temperature is in the range 150°-250°C, preferably 180°-220°.

**Patentansprüche**

1. Gasphasenverfahren zur Herstellung von HFC-125, umfassend die folgenden Schritte:

   - Einspeisen einer Mischung aus HCFC-124 und HFC-134a auf einen heterogenen Katalysator;

   - Abtrennen des HFC-125 von den anderen ReaktionsProdukten durch Destillation;

   wobei:
   der heterogene Katalysator aus Fluorierungskatalysatoren für halogenierte Kohlenwasserstoffe ausgewählt ist.

2. Verfahren nach Anspruch 1, wobei das Molverhältnis der Reaktanten, 134a/124, über 0,6 liegt.

3. Verfahren nach den Ansprüchen 1-2, wobei der Fluorierungskatalysator ausgewählt ist aus Oxiden, Oxyfluoriden, Oxychloriden, Chrom-, Cobalt-, Nickelfluoriden oder Mischungen davon.

4. Verfahren nach Anspruch 3, wobei der Fluorierungskatalysator ausgewählt ist aus Oxiden, Oxyfluoriden, Oxychloriden, dreiwertigen Chromfluoriden oder Mischungen davon, gegebenenfalls auf $AlF_3$, fluoriertem Aluminiumoxid oder Aktivkohle als Träger.

5. Verfahren nach Anspruch 4, wobei der Fluorierungskatalysator $Cr_2O_3$ ist, gegebenenfalls auf $AlF_3$ als Träger.

6. Verfahren nach den Ansprüchen 1-5, wobei der Fluorierungskatalysator vor Reaktionsbeginn durch Erhitzen (300°-400°C) in einem Inertgasstrom und anschließende Behandlung mit wasserfreiem (reinem oder verdünntem) HF bei gleicher Temperatur getrocknet wird.

7. Verfahren nach den Ansprüchen 1-6, wobei das Molverhältnis der eingespeisten Reaktanten, 134a/124, im Bereich von 0,6-2 liegt.

8. Verfahren nach den Ansprüchen 1-7, wobei die Reaktionstemperatur im Bereich von 150°-250°C, vorzugsweise 180°-220°C liegt.

**Revendications**

1. Procédé en phase gazeuse pour la production de HFC-125, comprenant les étapes suivantes :

- alimentation d'un mélange de HCFC-124 et de HFC-134a sur un catalyseur hétérogène ;
- séparation du HFC-125 des autres produits de réaction par distillation,

  dans lequel
  le catalyseur hétérogène est choisi parmi les catalyseurs de fluoration d'hydrocarbures halogénés.

2. Procédé selon la revendication 1, dans lequel le rapport molaire des réactifs 134a/124 est supérieur à 0,6.

3. Procédé selon les revendications 1 - 2, **caractérisé en ce que** le catalyseur de fluoration est choisi parmi les oxydes, les oxyfluorures, les oxychlorures, les fluorures du chrome, du cobalt, du nickel ou des mélanges de ceux-ci.

4. Procédé selon la revendication 3, dans lequel le catalyseur de fluoration est choisi parmi les oxydes, les oxyfluorures, les oxychlorures, les fluorures du chrome trivalent ou des mélanges de ceux-ci, éventuellement supportés sur du $AlF_3$, de l'alumine fluorée ou du carbone.

5. Procédé selon la revendication 4, dans lequel le catalyseur de fluoration est le $Cr_2O_3$, éventuellement supporté sur du $AlF_3$.

6. Procédé selon les revendications 1 - 5, dans lequel le catalyseur de fluoration est séché avant que la réaction démarre par chauffage (300°C-400°C) dans un flux de gaz inerte, suivi d'un traitement avec du HF anhydre (pur ou dilué) à la même température.

7. Procédé selon les revendications 1 - 6, dans lequel le rapport molaire 134a/124 des réactifs alimentés est dans la plage de 0,6-2.

8. Procédé selon les revendications 1 - 7, dans lequel la température de réaction est dans la plage de 150°C à 250°C, de préférence de 180°C à 220°C.